(19) Europäisches Patentamt
European Patent Office
Office européen des brevets

(11) EP 3 331 614 B1

(12) **EUROPEAN PATENT SPECIFICATION**

(45) Date of publication and mention of the grant of the patent:
**11.03.2020 Bulletin 2020/11**

(21) Application number: **16770827.0**

(22) Date of filing: **08.09.2016**

(51) Int Cl.:
*A61Q 1/12* (2006.01) *A61Q 1/10* (2006.01)
*A61K 8/81* (2006.01) *A61K 8/88* (2006.01)

(86) International application number:
**PCT/US2016/050649**

(87) International publication number:
**WO 2017/044546 (16.03.2017 Gazette 2017/11)**

(54) **FILM FORMING COMPOSITION**

FILMBILDENDE ZUSAMMENSETZUNG

COMPOSITION FILMOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2015 US 201562215199 P**

(43) Date of publication of application:
**13.06.2018 Bulletin 2018/24**

(73) Proprietor: **Noxell Corporation**
**Hunt Valley, Maryland 21030 (US)**

(72) Inventors:
- **ZHU, Yong**
  **Cincinnati, Ohio 45202 (US)**
- **GUAY, Gordon, Gerald**
  **Joppa, Maryland 21085 (US)**

(74) Representative: **Hanna Moore + Curley**
**Garryard House**
**25/26 Earlsfort Terrace**
**Dublin 2, D02 PX51 (IE)**

(56) References cited:
**WO-A1-2009/086338**
**WO-A1-2011/133511**
**US-A1- 2013 164 241**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 331 614 B1

## Description

FIELD OF THE INVENTION

The present application is directed, generally, to film forming compositions as defined in the appended claims. . These film forming compositions can be useful in a variety of applications, including use as a mascara product, a hair styling product, a skin care product, a skin foundation product, a lip product, and a nail care product. Specifically, there is disclosed a mascara formula that provides an improved curl benefit to the eyelashes of a user when the mascara is applied.

BACKGROUND OF THE INVENTION

Mascara compositions and eyelash tools have been used for many years to increase the thickness, length, curling, lifting and overall aesthetic appearance of human eyelashes. For curling benefits, most currently marketed mascara formulations are based on providing some shape retention after the lash shape has been altered by mechanical tools, such as curlers and/or mascara brushes with formulation. See e.g. WO 2009/086338 A1 (OREAL [FR]; BUI HY SI [US]; KANJI MOHAMED [US]; ATIS BALANDA [US]; HAL) 9 July 2009 (2009-07-09).

However, the two-step process of using a curler followed by mascara is cumbersome and time-consuming with some consumers choosing not to use curlers due to the fear of harming the lashes, skin or eyes. In addition, mechanical curlers often damage lash cuticles causing cuticle fracturing and chipping and over time the damage can be even worse resulting in hair fracturing leading the lash breakage. They physically crush the lash to alter the shape by plastically deforming the lash. The lashes can be damaged as a result. Through this process, the cortex of the hair can be exposed, resulting in breakage over time. This problem is exacerbated if the curler is used improperly. An example of improper use is repeated use of the curler.

Another method frequently used by consumers is to curl their lashes directly using mascara brushes with mascara formulas on the brush, allowing the user to curl up their lashes and apply mascara in one step. However this method is less effective than the two step curler method. The mascara compositions themselves do not generate any curl. As the result, the lash curl effect is minimal and the appearance of lengthening is small.

Eyelash curl or lift can also be generated by heat in a combination with curlers or other mechanical tools. The shape generated by mechanical forces can be fixed by heat, providing a more durable lash curl. However, since the heat used for this purpose must be sufficiently high to soften the lashes and facilitate internal structure arrangement, such as H-bonding rearrangement, the method can easily damage eyelashes and usually takes a longer time. In addition, this method requires heightened attention and skill by the consumer to avoid hurting nearby skin.

Accordingly it would be desirable to provide a mascara product that delivers more effective lash curl and lift, without difficulty in applications and damaging the lash.

BRIEF DESCRIPTION OF THE DRAWINGS

Figure 1 is an image of a Leneta card with lines indicating how to determine length for a contraction percentage measurement.

Figure 2 is an image of an evenly contracted Leneta card with lines indicating how to determine length for a contraction percentage measurement.

Figure 3 is an image of a non-evenly contracted Leneta card with lines indicating how to determine length for a contraction percentage measurement.

Figure 4 is an image of a contracted Leneta card that has coiled with lines indicating how to determine length for a contraction percentage measurement.

Figure 5 is an image of false lashes demonstrating a curl effect by comparing the lashes before polymer application and after polymer application

SUMMARY OF THE INVENTION

In order to provide a solution to the aforementioned problems, disclosed herein is a specific polymer combination system, as defined in claim 1, that provides a more durable, high strength contraction force and fast drying profile when applied to a substrate. When used as mascara, this composition curls and lifts the eyelash on the side of the lashes it is applied to, producing a lengthening appearance. The composition also solidifies quickly to maintain the shape of the styled lash for a longer period of time and make the styled lash more durable. The present invention comprises a film

forming composition comprising a first non-crosslinking polyamide/polyacrylate random copolymer comprising at least one monomer including vinyl caprolactam monomers, vinylpyrrolidone monomers and acrylamide monomers; (meth)acrylate monomers; monomers having at least one carboxylic functional group selected from the group consisting of carboxylic esters, carboxylic acids and their salts, or precursors of carboxylate functions, and mixtures thereof; and monomers having at least one amine functional group including primary, secondary and tertiary amines. The film forming composition also comprises a second non-crosslinking polyamide random copolymer comprising at least one monomer including vinyl caprolactam monomers, vinylpyrrolidone monomers, and (meth)acrylamide monomers; at least one quaternary ammonium containing monomer; and monomers having at least one amine functional group including primary, secondary, and tertiary amines. Preferably, the film forming composition is essentially free (less than about 0.5 weight %) of glycerin.

The first and second copolymers of the film forming composition comprise from 40 % to 99 % indentical monomers. In another embodiment, the first and second copolymers have from about 60% to about 99% of the same monomers. In yet another embodiment, the first and second copolymers have from about 70% to about 99% of the same monomers.

The structure similarity of the first and second copolymers based on monomer content in polymer compositions can be calculated based on theoretical syntheses or analyses of final products in synthetic processes, and can also be estimated using MASS, NMR, XPS, and FTIR tools.

In one embodiment, the second copolymer has one or more quaternary ammonium or other cationic side chains and further, the second copolymer comprises from about 0.1 to about 45 percent of the quaternary ammonium or other cationic side chains. In another embodiment, the second copolymer has one or more quaternary ammonium or other cationic side chains and further, the second copolymer comprises from about 1 to about 10 percent of the quaternary ammonium or other cationic side chains.

In one embodiment, the first copolymer has one or more carboxylic or other anionic side chains and further, the first copolymer comprises from about 0.1 to about 45 percent of said carboxylic or other anionic side chains. In another embodiment, the first copolymer has one or more carboxylic or other anionic side chains and further, the first polymer comprises from about 1 to about 10 percent of said carboxylic or other anionic side chains. In one embodiment of the film forming composition, the ratio of the first copolymer to said second copolymer is from about 1:50 to about 50:1. In another embodiment, the ratio of the first copolymer to the second copolymer is from about 1:10 to about 10:1. In yet another embodiment, the ratio of the first copolymer to the second copolymer is about 1:3 to about 3:1. In another embodiment, the ratio of the first copolymer to the second copolymer is about 1:1.

In one embodiment of the film forming composition, the first and second copolymers have a combined total polymer solid level greater than about 1% by weight of the composition (w/w). In one embodiment of the film forming composition, the first and second copolymers have a combined total polymer solid level greater than about 2% by weight of the composition (w/w). In another embodiment, the first and second copolymers have a combined total polymer solid level greater than about 5% by weight of the composition (w/w). In another embodiment, the first and second copolymers have a polymer solid level from about 2% to about 50% by weight of the composition (w/w). In yet another embodiment, the first and second copolymers have a polymer level from about 5% to about 30% by weight of the composition (w/w).

The present invention also encompasses a water based system comprising the film forming composition. Water based systems include water phase systems or two-phase systems such as solid in water suspension, water-in-oil/silicone or oil/silicone-in-water systems.

In one embodiment, the film forming composition produces a desired contraction when applied to a double coated opacity chart, such as Form 2A (supplied by Leneta Company referred here as Leneta card). When the composition is applied to a Leneta card, the card has a minimum contraction of 10% with minimum synergy of 120% (as described by the "Contraction Test" method below) when the Leneta card is kept at a temperature in the range of 22°C to 28°C and at a relative humidity in the range of 40% to 50% to measure the contraction.

In another embodiment, the film forming composition produces a fast dry kinetics when measured using a "Weight Loss Test" method described below. When the composition is applied to a flat hard substrate, such as a glass microscope slide, the film has a shorter dry time, having a Dry Speed (i.e., time required to reach 90% total weight loss) of less than 10 minutes with a Dry Speed synergy of at least about 110%. Dry Speed synergy is calculated according to the method disclosed in the Examples (below).

The film forming composition can be used in a variety of applications, including as a mascara formulation, a hair styling product, a skin care product, a skin foundation product, a lip product, and a nail care product.

## DETAILED DESCRIPTION OF THE INVENTION

All percentages are by weight of the personal-care composition, unless otherwise specified. All ratios are weight ratios, unless specifically stated otherwise. All numeric ranges are inclusive of narrower ranges; delineated upper and lower range limits are interchangeable to create further ranges not explicitly delineated. The number of significant digits

conveys neither limitation on the indicated amounts nor on the accuracy of the measurements. Unless otherwise stated or prescribed, all measurements are understood to be made from about 22-28°C and at ambient conditions, where "ambient conditions" means conditions under about one atmosphere of pressure and at about 40-50% relative humidity.

Definitions

"Keratinous tissue," means keratin-containing tissue layers disposed as the outermost protective covering of mammals which includes, but is not limited to, skin, hair, and nails.'

"Mascara" and "mascara composition" mean a liquid, semi-solid, or solid cosmetic composition that is applied to eyelashes to provide an aesthetic benefit or change in appearance such as, the appearance of a color change, a volume change, and/or a length change. Mascara may also be applied to periorbital areas, eyelids and/or eyebrows. The present mascara compositions are formulated for topical application to mammalian keratinous tissue for use in cosmetic products. The methods of using mascara compositions are also included within the meaning of mascara composition.

"Non-crosslinking polyamide/polyacrylate random copolymer" means any non-crosslinking amide and acrylate monomer containing random copolymer that include the amide and acrylate monomers as side chains. An example of such amide and acrylate monomer containing copolymers is Styleze 2000 as supplied by ASI.

"Non-crosslinking polyamide random copolymer" means any non-crosslinking amide monomer containing random copolymer that includes the amide monomers as side chains. An example of such amide monomer containing copolymers is Aquastyle 300 as supplied by ASI.

"Water-soluble, film-forming polymers" are defined herein to mean polymers which are soluble or dispersible in water, water-cosolvent mixtures (such as ethanol/water), pH adjusted water, and/or tempered solutions of the above to facilitate solubilization or dispersion of the polymers.

Film-Forming Composition

Disclosed herein is a specific polymer combination system that provides a more durable, high strength contraction force and fast dry profile when applied to substrate. When used as mascara and applied to the lashes, this produces desired eyelash curl and lift to gain lengthening appearance, and meanwhile undergoes fast solidification to keep the lash styling longer and durable. The present invention comprises a film forming composition comprising a first non-crosslinking polyamide/polyacrylate random copolymer comprising at least one amide monomer including vinyl caprolactam monomers, vinylpyrrolidone monomers and acrylamide monomers; (meth)acrylate monomers; monomers having at least one carboxylic functional group selected from the group consisting of carboxylic esters, carboxylic acids and their salts, or precursors of carboxylate functions, and mixtures thereof; and amine functional groups including primary, secondary and tertiary amines. The film forming composition also comprises a second non-crosslinking polyamide random copolymer comprising at least one amide monomer including vinyl caprolactam monomers, vinylpyrrolidone monomers, and (meth)acrylamide monomers; at least one quaternary ammonium containing monomer; and amine functional groups including primary, secondary, and tertiary amines.

Preferably, the film forming composition is essentially free (less than about 0.5 weight %) of glycerin.

Amide monomers that are useful in the present invention include amide monomers with open-chain organic amide functional groups and derivatives having general formula of

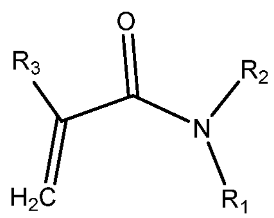

wherein R1 is H, or C1-C5 alkyl or alkylene, R2 is H, or C1-C18 alkyl or alkylene

R1 and R2 alkyl and alkylene are independently linear or branched

R1 and R2 alkyl and alkylene independently have either no functional groups linked, or linked to other functional groups, including amides, amines, quaternized amines, acid-esters/acids/salts, or their derivatives
wherein R3 is H, or C1-C5 alkyl linear or branched.

In one embodiment, the preferred amides include acrylamides and methacrylamides.

Commercially available examples of such amide monomers include the monomers in Styleze W, Styleze CC-10, AquaStyle 300 (PQ69), Aquaflex SF-40, ViviPrint 141, Conditioneze NT-20 all commercially available from Ashland Specialty Ingredients (ASI); and Ultrahold Strong, Luviset Clear, Luviquat Supreme (PQ68) all available from BASF. Other examples of this type of polymers can be found in Personal Care Product Consult Database (PCPC).

Amide monomers that are useful in the present invention include amide monomers with cyclic amide functional groups and derivatives having general formula of

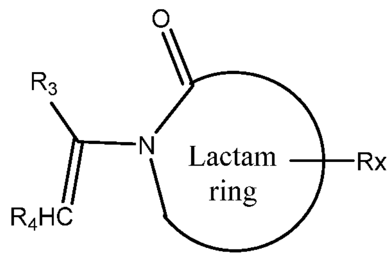

Cyclic amides N-cyclic(Rx) are lactams, having 5 to 7 member rings (e.g. vinylpyrrolidones, vinyl caprolactams).

The lactam rings containing alkyl Rx group or groups linked to one or more C atoms on the ring Rx group or groups are independently H or C1-C30 linear or branched
wherein R3, is H, or C1-C5 alkyl linear or branched,
wherein amide monomers may contain one or more types of amide functional groups (e.g. open and/or cyclic amides, vinylpyrrolidones and/or vinyl caprolactams).

Commercially available examples of such amide monomers include the monomers in Copolymer 845, 937 and 958, Advantage LCA, LCE and S, PVP/VA (W635, 735), Gafquat, Aquaflex SF-40, Styleze W, Aquastyle 300, ViviPrint 141, Conditioneze NT-20, Styleze CC-10 all available from ASI; and Luviquat Supreme, Luviquat UltraCare, Luviquat Hold, Luviquat PQ11, Luviquat HM552, Luviquat Style, Luviquat FC, Luviquat Excellence, Luviset Clear all available from BASF. Other examples of this type of polymers can be found in Personal Care Product Consult Database (PCPC).

Monomers that are useful in the present invention include amine monomers with functional groups and derivatives having general formula of

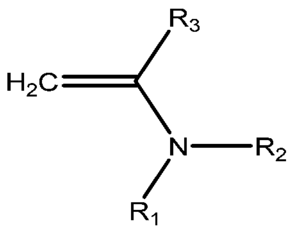

or

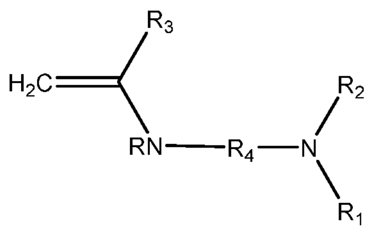

wherein $R_1$ is H, or C1-C5 alkyl or alkylene, $R_2$ is H, or C1-C18 alkyl or alkylene, and $R_4$ is C1-C10 alkyl or alkylene
$R_1$, $R_2$, and $R_4$ alkyl or alkylene are independently linear or branched
$R_1$, $R_2$, and $R_4$ alkyl or alkylene independently have either no functional groups linked, or linked to other functional groups, including amides, imides, amines, quaternized amines, and acid-esters/acids/salts, or their derivatives
wherein the amine monomers are primary, secondary and/or tertiary amines wherein $R_3$ is H, or C1-C5 alkyl linear or branched
wherein NR is a secondary or tertiary amine

Commercially available examples of such amine monomers include the monomers in Copolymer 845, 937 and 958, Advantage LCA, LCE and S, Gafquat, Aquaflex SF-40, Styleze W, Aquastyle 300, ViviPrint 141, Aquaflex XL-30, Styleze CC-10 all available from ASI; and Luviquat Supreme from BASF. Other examples of this type of polymers can be found in Personal Care Product Consult Database (PCPC).

Monomers that may be useful in the present invention include amine or ether monomers with quaternary

ammonium functional groups and derivatives having general formula of

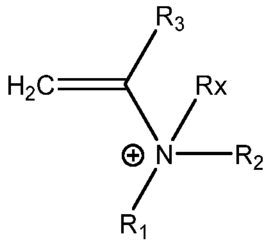

or

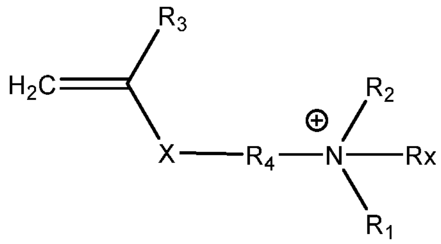

wherein $R_1$ and $R_2$ are independently H, or C1-C5 alkyl or alkylene, and $R_4$ is C1-C10 alkyl or alkylene
$R_1$, $R_2$, and $R_4$ alkyl or alkylene are independently linear or branched
$R_1$, $R_2$, and $R_4$ alkyl or alkylene independently have either no functional groups linked, or linked to other functional groups, including amides, imides, amines, quaternized amines, and acid-esters/acids/salts, or their derivatives
wherein the amine functional groups are primary, secondary, and tertiary amines; open and cyclic amines
wherein Rx is C1-C18 alkyl linear or branched
wherein $R_3$ is H, or C1-C5 alkyl linear or branched
wherein X is O or NR
where NR is a secondary or tertiary amine.

Commercially available examples of copolymers containing such monomers include Polyquaternium-5, -11, -14, -19, -22, -28, -37, -46, -47, -51, -55, -69, -87 (all available from BASF).

Monomers that may be useful in the present invention include amide or acrylate monomers with quaternary ammonium functional groups and derivatives having general formula of

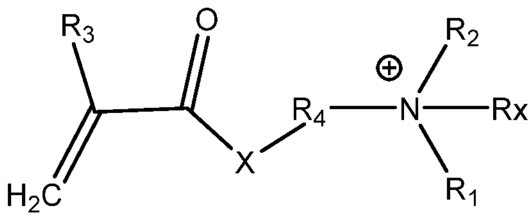

wherein $R_1$ and $R_2$ are independently H, or C1-C5 alkyl or alkylene, and $R_4$ is C1-C10 alkyl or alkylene
$R_1$, $R_2$, and $R_4$ alkyl or alkylene are independently linear or branched
$R_1$, $R_2$, and $R_4$ alkyl or alkylene independently have either no functional groups linked, or linked to other functional groups, including amides, imides, amines, quaternized amines, and acid-esters/acids/salts, or their derivatives
wherein the amine functional groups are primary, secondary, and tertiary amines; open and cyclic amines
wherein Rx is C1-C18 alkyl linear or branched
wherein $R_3$ is H, or C1-C5 alkyl linear or branched
wherein X is O or NR
wherein NR is a secondary or tertiary amine.

Commercially available examples of copolymers containing such monomers include Polyquaternium-4, -5, -7, -8, -9, -11, -12, -13, -18, -28, -33, -36, -37, -45, -47, -49, -52, -53, -55, - 63, -64, -68, -69, -85, -89, -91, -109, and others as described PCPC.

Monomers that may be useful in the present invention include monomers with carboxylic acid, salt and ester functional groups and derivatives having general formula of

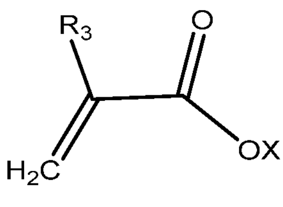

or

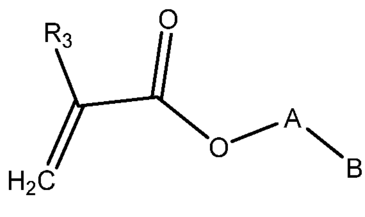

wherein X is H, or ionizable metal ions, and A is C1-C8 alkyl or alkylene group
the A alkyl or alkylene is linear or branched
the B is H, amide, imide, amine, quaternized ammonium, or acid-ester/acid/salt, or their derivatives
wherein the amine functional groups are primary, secondary, and tertiary amines; open and cyclic amines
wherein $R_3$ is H, or C1-C5 alkyl linear or branched

In one embodiment, preferred monmers include: acrylates, methacrylates, acrylic acids and their salts, methacrylic acids and their salts. The carboxylate monomers may include precursors of carboxylate functions, such as tert-butyl (meth)acrylates, alkyl-2-amino ethyl esters of (meth)acrylates which give rise to carboxylic functions by hydrolysis (under more stressed pH, temperature conditions, in the presence of catalysts, or other approaches).

Commercially available examples of copolymers containing such monomers include Advantage Plus, LCA, LCE and S, W635 and 735, Copolymer 845, 937 and 958, Aquaflex XL-30, PVP/VA E-735, E-635, E-535 and W-735, Gafquat, Allianz OPT from ASI; Luviquat PQ11, UltraHold Strong, Luviset Shape, Luviflex Soft, Cosmedia SP from BASF. Other examples of this type of polymers can be found in Personal Care Product Consult Database (PCPC).

The ester/acid/salt/anhydride functional groups may contain one or more types of esters/acids/salts/anhydrides (e.g. esters, acids and/or salts).

The first and second copolymers of the film forming composition comprise from 40 % to 99 % identical monomers. In another embodiment, the first and second copolymers have from about 50% to about 99% of the same monomers. In another embodiment, the first and second copolymers have from about 60% to about 99% of the same monomers. In yet another embodiment, the first and second copolymers have from about 70% to about 99% of the same monomers.

Polymer structure similarity is a useful criterion for achieving the present invention's unexpected high contraction and improved dry speed synergy. Another criterion is the charge density of the polymers, which can be useful for delivering the unexpected high contraction and fast dry synergy performances.

The table and description below illustrates polymer structure similarity or dissimilarity based on monomer contents:

| Polymers from BASF | % vinylcaprolactam | % vinylpyrrolidone | % Acrylate derivative | % Quaternary ammonium |
|---|---|---|---|---|
| Luviquat Supreme | 0 | 55 | 29 | 6 |
| Luviquat UltraCare | 0 | 80 | 0 | 20 |
| Luviquat Hold | 0 | 40 | 0 | 10 |
| Luviquat PQ11 | 50 | 67 | 0 | 0 |
| Luviquat Style | 0 | 55 | 0 | 45 |
| Luviquat FC370 | 0 | 70 | 0 | 30 |
| Luviquat FC550 | 0 | 50 | 0 | 50 |

Here it can be seen that Luviquat Supreme and Luviquat UltraCare have 61% similarity (55% shared vinylpyrrolidone + 6% shared Quaternary ammonium), while Luviquat Hold and Luviquat PQ11 have 40% similarity (40% shared vinylpyrrolidone only). Likewise, the structure similarity of other BASF polymers listed in the table can also be estimated.

The figures below show structures of polymers Advantage S and Aquastyle 300 from ASI. The monomer contents of Aquastyle 300 are cited as preferred ranges from the patent US 6,852,815 by ISP (now ASI), and the monomer contents of Advantage S are estimated based on spectra similarity as evaluated by MASS, NMR XPS, and/or FTIR.

Advantage S

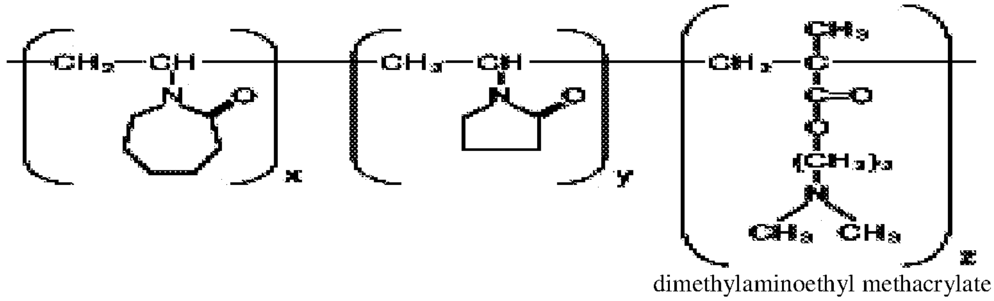

| | | |
|---|---|---|
| vinyl caprolactam (VCL) (55 - 75%) | vinylpyrrolidone (VP) (20 - 40%) | dimethylaminoethyl methacrylate (DMAEMA) (0.1 - 15%) |

Aquastyle 300

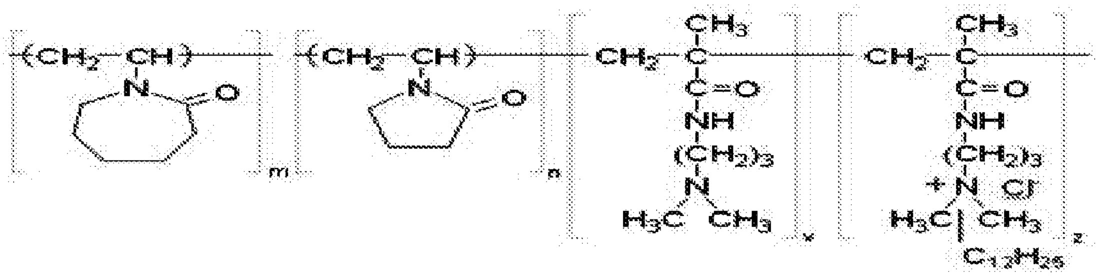

| | | | |
|---|---|---|---|
| vinyl caprolactam (VCL) (55 - 75%) | vinylpyrrolidone (VP) (20 - 40%) | dimethylaminopropyl methacrylamide (DMAPMA) (0.1 - 5%) | methacryloylaminopropyl lauryldimethyl ammonium chloride (MAPLDMAC) (1 -10%) |

where Advantage S and Aquastyle 300 are estimated based on the monomer contents to have a minimum structure similarity of about 75% (at least 55% shared vinyl caprolactam monomers and at least 20% shared vinylpyrrolidone monomers).

In one embodiment, the preferred second copolymer comprises from about 0.1 to about 45 percent of quaternary ammonium containing monomers. In another embodiment, the preferred second copolymer comprises from about 1 to about 10 percent of quaternary ammonium containing monomers.

In one embodiment, the preferred first copolymer comprises from about 0.1 to about 40 percent of carboxylic functional groups. In another embodiment, the preferred first copolymer comprises from about 1 to about 10 percent of carboxylic functional groups.

In one embodiment of the film forming composition, the ratio of the first copolymer to said second copolymer is from about 1:50 to about 50:1. In another embodiment, the ratio of the first copolymer to the second copolymer is from about 1:10 to about 10:1. In yet another embodiment, the ratio of the first copolymer to the second copolymer is about 1:3 to about 3:1. In another embodiment, the ratio of the first copolymer to the second copolymer is about 1:1.

In one embodiment of the film forming composition, the first and second copolymers have a combined polymer solid level greater than about 1% by weight of the composition (w/w). In another embodiment of the film forming composition, the first and second copolymers have a combined total polymer solid level greater than about 2% by weight of the composition (w/w). In another embodiment, the first and second copolymers have a combined total polymer solid level greater than about 5% by weight of the composition (w/w). In another embodiment, the first and second copolymers have a combined polymer solid level from about 2% to about 50% by weight of the composition (w/w). In yet another embodiment, the first and second copolymers have a combined polymer solid level from about 5% to about 30% by weight of the composition (w/w).

In one embodiment of the film forming composition, the first and second copolymers are random polymers and include water soluble polymers. The water soluble polymers have molecular weight MW of greater than about 10,000. In another embodiment, the water soluble polymers have molecular weight MW from 20,000 to 4,000,000. In another embodiment, the water soluble polymers have molecular weight MW from 50,000 to 2,000,000.

## Charge Density

The copolymers in the combinations in the invention are characterized as cationic (containing quaternary ammonia or other permanent cationic ions), pseudo-cationic or pH dependent cationic (containing primary, secondary and tertiary amines,), anionic (containing carboxylic ions or other anionic ions), pseudo-anionic or pH dependent anionic (containing carboxylic acid, carboxylic ester or hydrolysable/degradable ester), or precursor of anionic functions, amphoteric, or zwitterionic in charges.

The charge density (cationic or anionic) of the polymers in this invention is expressed in milliequivalent per gram (mEq/g), defined theoretically as the number of the equivalent charged groups per gram of polymer mass. The theoretical charge density may be determined based on the contents of charged monomers in the polymers.

The apparent or relative charge density or surface charge density of water soluble or water dispersible polymers and/or particles with hydrodynamic diameter or mean particle size of 10-1000nm in size may be determined or supported by zeta-potential, streaming potential, or streaming current.

In one embodiment, the second polymer has a positive charge density from greater than 0.0 to about 1.0 milliequivalents/gram (mEq/g) based on quaternized amine monomer contents. In another embodiment, the second polymer has a positive charge density from greater than 0.0 to about 0.5 mEq/g based on quaternized amine monomer contents. In another embodiment, the second polymer has a positive charge density from greater than 0.0 to about 0.1 mEq/g based on quaternized amine monomer contents.

In another embodiment, the first polymer has a negative charge density from less than 0.0 to about -1.0 mEq/g based on anionic monomer contents. In another embodiment, the first polymer has a negative charge density from less than 0.0 to about -0.5 mEq/g based on anionic monomer contents. In another embodiment, the first polymer has a negative charge density from less than 0.0 to about -0.1 mEq/g based on anionic monomer contents.

## Contraction

In one embodiment, the film forming composition produces a desired contraction when applied to a Leneta card. When the composition is applied to a Leneta card, the card has a minimum contraction of 10% with minimum synergy of 120% (as described by the "Contraction Test" method below) when the Leneta card is kept at a temperature in the range of 22 to 28°C and at a relative humidity in the range of about 40% to 50% to measure the contraction.

## Fast Dry

In another embodiment, the film forming composition produces fast dry kinetics when measured using a "Weight Loss Test" method described below. When the composition is applied to a flat hard substrate, such as glass microscope slide, the film has a shorter dry time, as determined by having a Dry Speed (i.e., time required to reach 90% total weight loss) of less than 10 minutes with a Dry Speed synergy of at least about 110%.

## Dry films and Powders

In one embodiment, the film forming compositions of the present invention may be used as a dry film or as a powder. These dry forms of the film forming compositions can be applied to a wetted surface or wetted after they are applied to a surface to deliver mechanical benefits to lashes and/or skin. The shrinkage benefits that are obtained from the film forming compositions of the present invention are capable of being rejuvenated with water exposure, enabling benefits to cycle from wet to dry to wet to dry while still maintaining the technical polymer benefit.

For lashes, dry film and/or powder forms of the film forming compositions may be placed on wetted lashes or lashes that are wetted after film deposition to produce lash lift and curl. For skin, powder and/or film forms can be applied to pre or post wetted areas of skin to enable skin tensing effects to impact fine lines and wrinkles. In another embodiment, the film forming compositions of the present invention may be used as a combination of dry film, powder and/or wet formulation.

In one embodiment, the dry film and powders of the present invention can be used for targeted applications on lashes or skin. For lashes, dry formulations could be applied and then wetted to produce different lash looks from lash lift, curl and lash lift and curl depending on where formulation has been applied. For example, the powder could be selectively applied to the lash only near the eyelid, the center of the lash, or the tip of the lash. For skin, dry formulations could be applied to wet skin, or applied and then wetted, on target areas, like the "crow's feet" area or the forehead, to enable targeted skin tensing effects to impact fine lines and wrinkles.

In one embodiment, for targeted application on lashes, a modified eyelash curling device could be used. In such a modified device, the rubber-like strip is replaced with a powder or film delivery mechanism. In another embodiment, the delivery mechanism is a trough filled with powder.

Products

The film forming composition of the present invention can be used in a variety of applications, including as a mascara formulation, a hair styling product, a skin care product, a skin foundation product, a lip product, a nail care product, and a kit. The present invention also encompasses a water based system comprising the film forming composition.

The compositions disclosed herein may be used in many end-use applications. Examples include (but are not limited to) a water phase suspension, an oil in water emulsion, a water in oil emulsion, a silicone in water emulsion, a water in silicone emulsion, a Pickering emulsion, and/or an oil phase suspension, and/or kits.

Carrier and/or Oil

When the film forming composition is incorporated into a mascara formulation, the mascara may include a carrier to help deliver the desired mascara components (e.g., the film former, pigments, etc.) to the eyelash or eyelid. In certain embodiments, the mascara composition may include a volatile carrier that quickly volatilizes from the surface of the eyelashes or eyelid, leaving the desired components behind. The volatile carrier may be present at 2% to 85%, 10% to 80%, or even 20% to 70% by weight based on the weight of the composition. Nonlimiting examples of suitable volatile carriers include volatile hydrocarbons, volatile alcohols, volatile silicones, and mixtures thereof.

Hydrocarbon oils suitable for use as a carrier in the present mascara compositions include those having boiling points in the range of 60-260°C, such as hydrocarbon oils having a carbon chain length of from C8 to C20 (e.g., C8 to C20 isoparaffins). Particularly suitable examples of isoparaffins include those selected from the group consisting of isododecane, isohexadecane, isoeicosane, 2,2,4-trimethylpentane, 2,3-dimethylhexane and mixtures thereof. Isododecane is available from Presperse under the brand name Permethyl 99A. Alcohols suitable for use may include $C_1$ - $C_4$ monoalcohols, such as ethyl alcohol and isobutyl alcohol.

A volatile silicone fluid may also be used as a carrier herein. Suitable volatile silicone fluids include dimethicone, trimethicone, and cyclomethicones. Nonlimiting examples of commercially available volatile silicones include 244 Fluid, 344 Fluid and 245 Fluid, and/or 345 Fluid from Dow Corning Corporation.

Oils typically used in cosmetics include those selected from the group consisting of polar oils, non-polar oils, volatile oils, non-volatile oils and mixtures thereof. These oils may be saturated or unsaturated, straight or branched, aliphatic or aromatic hydrocarbons. Preferred oils include non-polar volatile hydrocarbons including isodecane (such as Permethyl-99A®, available from Presperse Inc.) and the $C_7$-$C_8$ through $C_{12}$-$C_{15}$ isoparaffins (such as the Isopar® Series available from Exxon Chemicals).

Non-polar, volatile oil may be included in the cosmetic composition to impart desirable aesthetic properties (e.g., good spreadability, non-greasy and/or tacky feel, quick drying to allow pigment particles to set on skin) to the present cosmetic composition. Non-polar, volatile oils suitable for use herein include silicone oils; hydrocarbons; and mixtures thereof. The non-polar, volatile oils may be either saturated or unsaturated, have an aliphatic character and be straight or branched chains or even contain alicyclic or aromatic rings. Examples of suitable non-polar, volatile hydrocarbons for use herein include polydecanes such as isododecane and isodecane (e.g., Permethyl-99A which is available from Presperse Inc.), dodecanes and tetra dodecanes (such as Parafol 12-97 and Parafol 14 from Sasol), and the C7-C8 through C12-C15 isoparaffins (such as the Isopar Series available from Exxon Chemicals). Exemplary non-polar, volatile liquid silicone oils are disclosed in U.S. Pat. No. 4,781,917. Additionally, a description of various volatile silicone oils may be found in Todd et al., "Volatile Silicone Fluids for Cosmetics", Cosmetics and Toiletries, 91:27-32 (1976). Particularly suitable volatile silicone oils include cyclic volatile silicones corresponding to the formula:

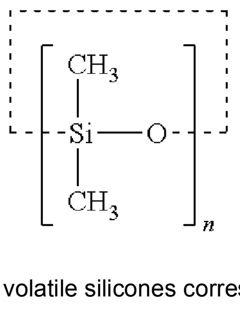

wherein n is from about 3 to about 7; and linear volatile silicones corresponding to the formula:

$$(CH_3)^3Si\text{-}O\text{-}[Si(CH_3)^2\text{-}O]^m\text{-}Si(CH_3)^3)^3$$

wherein m is from about 0 to about 7. Linear volatile silicone oils generally have a viscosity of less than about 5 centistokes

at 25° C., whereas the cyclic silicones have viscosities of less than about 10 centistokes at 25° C. Examples of suitable volatile silicone oils include cyclomethicones of varying viscosities, e.g., Dow Corning 200, Dow Corning 245, available from Dow Corning Corp.); SF-1204 and SF-1202 Silicone Fluids (commercially available from Momentive Specialty Chemicals), and SWS-03314 (commercially available from Wacker Chemie AG.). In addition, Caprylyl Methicone such as Dow Corning FZ3196 can be used. Other examples of non-polar, volatile oils are disclosed, for example, in Cosmetics, Science, and Technology, Vol. 1, 27-104 edited by Balsam and Sagarin, 1972.

Colorants

When the film forming composition is incorporated into a mascara formulation, the mascara may include colorants. Colorants suitable for use in the present mascara compositions include, but are not limited to, dyes, pigments, lakes, and mixture thereof. (e.g., organic or inorganic pigments and colorants approved for use in eye-area cosmetics by PCPC and/or the FDA.) Exemplary inorganic pigments include particles of iron oxides (e.g., yellow, brown, red, black), titanium dioxides, iron sulfides, ultramarines, chromium oxides (e.g., green) or other conventional pigments used in cosmetic formulations. Examples of organic pigments include D&C Black No. 2, D&C Black No. 3, FD&C Red No. 40, D&C Green No. 5, FD&C Blue No. 1, and FD&C Yellow No. 5. Examples of lake dyes include various acid dyes which are laked with aluminum, calcium or barium. Additional colorants for use herein include annatto, caramel, carmine, β-carotene, bismuth oxychloride, ferric ammonium ferrocyanide, ferric ferrocyanide, chromium hydroxides (e.g., green), guanine, mica, aluminum powder, bronze powder, copper powder, manganese violet, zinc oxide. Suitable colorants along with their chemical structure are described in, e.g., 21 C.F.R. Part 74 and in the PCPC Cosmetic Ingredient Handbook, (1988), published by the Cosmetics, Toiletry and Fragrances Association, Inc. Other colorants may also be used as they are developed and determined safe.

Encapsulated colorant microparticles having average diameters of 0.1 to 50 microns may be acceptable for use in mascara compositions. Suitable examples of encapsulated colorant microparticles are disclosed in copending U.S. Publication Nos. 20090263658 and 20090271932A1. The encapsulated colorant microparticles may comprise from 1 to 60% by weight of at least one colorant, for example 5% to 40% or even 6% to 25% by weight. Microencapsulated colorants may provide a more vibrant color to products used around the eye area, including eyelashes. The primary colors are understood to mean red, yellow and blue.

In one embodiment, mascara compositions according to the invention comprise from about 0.1 to about 70% by weight, for example from about 0.5 to about 50% by weight, and especially from about 1.0 to about 35% by weight based on the total weight of the composition, of a colorant. Colorants in the form of particles and/or encapsulants having average diameters of 0.1 to 50 microns may be acceptable for use in the present compositions. In another embodiment, the particles have average diameters of 0.1 to 10 microns. In another embodiment, the particles have average diameters of 0.1 to 5 microns. It may be desirable to select colorant particles with a diameter that is less than the thickness of the mascara composition dried-down film. The small size of the colorant particles may allow them to be fully encased in the dried film.

Thickeners

When the film forming composition is incorporated into a mascara formulation, the mascara may include thickeners. The mascara composition can be thickened or structured with colloidal particles and/or waxes.

Thickening agents that may be useful in the present invention include carboxylic acid polymers such as the carbomers (e.g., the CARBOPOL® 900 series such as CARBOPOL® 954 by Lubrizol). Other suitable carboxylic acid polymeric agents include copolymers of C10-30 alkyl acrylates with one or more monomers of acrylic acid, methacrylic acid, or one of their short chain (i.e., C1-4 alcohol) esters, wherein the crosslinking agent is an allyl ether of sucrose or pentaerytritol. These copolymers are known as acrylates/C10-30 alkyl acrylate crosspolymers and are commercially available as CARBOPOL® 1342, CARBOPOL® 1382, PEMULEN TR-1, and PEMULEN TR-2, from Lubrizol.

Additional suitable thickening agents include the polyacrylamide polymers and copolymers. An exemplary polyacrylamide polymer has the CTFA designation "polyacrylamide and isoparaffin and laureth-7" and is available under the trade name SEPIGEL 305 from Seppic Corporation (Fairfield, N.J.). Other polyacrylamide polymers useful herein include multi-block copolymers of acrylamides and substituted acrylamides with acrylic acids and substituted acrylic acids. Commercially available examples of these multi-block copolymers include HYPAN SR150H, SS500V, SS500 W, SSSA100H, from Lipo Chemicals, Inc., (Patterson, N.J.).

Other suitable thickening agents useful herein are sulfonated polymers such as the CTFA designated sodium polyacryloyldimethyl taurate available under the trade name Simulgel 800 from Seppic Corp. and Viscolam At 100 P available from Lamberti S.P.A. (Gallarate, Italy). Another commercially available material comprising a sulfonated polymer is Sepiplus 400 available from Seppic Corp.

Waxes may be useful as thickeners and/or as structuring agents including natural, synthetic, and surface

modified waxes, including cold water process wax (such as CPW brands by JEEN International Corp). Waxes are defined as lower-melting organic mixtures or compounds of high molecular weight, solid at room temperature and generally similar in composition to fats and oils except that they contain no glycerides. Some are hydrocarbons, others are esters of fatty acids and alcohols. Waxes useful in the present invention are selected from the group consisting of animal waxes, vegetable waxes, mineral waxes, various fractions of natural waxes, synthetic waxes, petroleum waxes, ethylenic polymers, hydrocarbon types such as Fischer-Tropsch waxes, silicone waxes, and mixtures thereof wherein the waxes have a melting point between 55° and 100°C and a needle penetration value, as measured according to the American standard ASTM D5, of 3 to 40 units at 25°C. The principle of the measurement of the needle penetration according to the standards ASTM D5 consists in measuring the depth, expressed in tenths of a millimeter, to which a standard needle (weighing 2.5 g and placed in a needle holder weighing 47.5 g, i.e. a total of 50 g) penetrates when placed on the wax for 5 seconds. Waxes are used at levels in order to provide sufficient bulk material that resists drying out after application, providing thickness to the lashes.

Waxes may be useful to maintain the film durability of the mascara composition. In some instances, the mascara composition may include from 0.1-15% wax In another embodiment, the mascara composition may include from 1-10% wax. In another embodiment, the mascara composition may include from 4-8% wax. In some instances, it may be desirable to include wax at an amount of less than 3.0%, for example, less than about 1.0% or even less than 0.1%, by weight, of wax and wax-like components. In some instances, the present mascara composition is free of wax.

Specific waxes that may be useful in the present invention include beeswax, lanolin wax, shellac wax (animal waxes); carnauba, candelilla, bayberry (vegetable waxes); ozokerite, ceresin, (mineral waxes); paraffin, microcrystalline waxes (petroleum waxes); polyethylene, (ethylenic polymers); polyethylene homopolymers (Fischer-Tropsch waxes); $C_{24-45}$ alkyl methicones (silicone waxes); and mixtures thereof. Most preferred are beeswax, lanolin wax, carnauba, candelilla, ozokerite, ceresin, paraffins, microcrystalline waxes, polyethylene, $C_{24-45}$ alkyl methicones, and mixtures thereof.

Clays may be useful to provide structure or thickening. Suitable clays can be selected, e.g., from montmorillonites, bentonites, hectorites, attapulgites, sepiolites, laponites, silicates and mixtures thereof. Suitable water dispersible clays include bentonite and hectorite (such as Bentone EW, LT from Rheox); magnesium aluminum silicate (such as Veegum from Vanderbilt Co.); attapulgite (such as Attasorb or Pharamasorb from Engelhard, Inc.); laponite and montrnorillonite (such as Gelwhite from ECC America); and mixtures thereof.

Disteardimonium hectorite is a suitable thickener to build structure/viscosity in the present mascara composition. This enables proper spreading/deposition across lashes, and ensures adequate stability/suspension of colorant particles in dispersion over time. It is preferable that the diameter of the disteardimonium hectorite is smaller than the thickness of the mascara composition dried-down film. The preferred diameter of the disteardimonium hectorite is less than 10 microns. The mascara compositions may comprise from about 1% to about 25% of suitable thickener such as disteardimonium hectorite, from about 2% to about 20%, or even from about 3% to about 15%. Suitable thickening agents also include cellulose and modified cellulosic compositions such as, carboxymethyl cellulose, hydroxyethylcellulose, cellulose acetate propionate carboxylate, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl substituted celluloses. In these polymers some portion of the hydroxy groups of the cellulose polymer are hydroyxalkylated (preferably hydroxyethylated or hydroxypropylated) to form a hydroxyalkylated cellulose which is then further modified with a C10-C30 straight chain or branched chain alkyl group through an ether linkage. Typically these polymers are ethers of C10-C30 straight or branched chain alcohols with hydroxyalkylcelluloses. Examples of alkyl groups useful herein include those selected from the group consisting of stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers is the material given the PCPC designation cetyl hydroxyethylcellulose, which is the ether of cetyl alcohol and hydroxyethylcellulose. This material is sold under the tradename Natrosol® CS Plus from ASI.

Actives

When the film forming composition is incorporated into a mascara formulation, the mascara may comprise a safe and effective amount of a biological, chemical, nutraceutical, or pharmaceutical active, or a combination thereof. Biological actives may include prostaglandins, antimicrobials, antibacterials, biocides, preservatives, proteins, amino acids, peptides, hormones, growth factors, enzymes (*e.g.*, glutathione sulphydryl oxidase, transglutaminase), therapeutics, oligonucleotides, genetic materials (*e.g.*, DNA, RNA), and combinations thereof. Chemical actives may include dyes, surfactants, sensates, hair conditioners, hair dyes, hair growth agents, hair styling gels, and combinations thereof. Nutraceutical actives may include proteins, preservatives, vitamins, food-additive materials, and combinations thereof. Pharmaceutical actives may include antibiotics, drugs, hair growth agents, and combinations thereof.

## Additional polymers

In addition to the first and second copolymers, the composition may also include additional polymers.

The mascara composition of the present invention may comprise additional water-soluble film forming polymers. In one embodiment, water-soluble, film forming polymers comprise from about 1% to about 50%, preferably from about 2% to about 40% and most preferably from about 3% to about 30% of the composition.

The additional polymers comprise polymers formed from monomers, said monomer derivatives, mixtures of said monomers, mixtures of said monomer derivatives, natural polymers and mixtures thereof. The film forming polymers disclosed herein also include chemically modified versions of the above disclosed polymers. Said monomers are selected from the group consisting of olefin oxides, vinyl pyrrolidone, vinyl caprolactam, vinyl esters, vinyl alcohols, vinyl cyanides, oxazilines, carboxylic acids and esters and mixtures thereof. Preferred vinyl pyrrolidone polymers are selected from the group consisting of polyvinylpyrrolidone, vinyl acetate/vinyl pyrrolidone copolymer and mixtures thereof. Preferred polyvinyl esters are selected from the group consisting of vinyl acetate/crotonic acid copolymer, vinyl acetate crotonic acid vinyl neodecanoate copolymer and mixtures thereof. Preferred vinyl alcohol polymers are selected from the group consisting of vinyl alcohol vinyl acetate, vinyl alcohol/poly(alkyleneoxy)acrylate, vinyl alcohol/vinyl acetate/poly- (alkyleneoxy)acrylate and mixtures thereof. Preferred olefin oxides are selected from the group consisting of polyethylene oxide, polypropylene oxide and mixtures thereof. Preferred polycarboxylic acids and their esters are selected from the group consisting of acrylates, acrylates/octylacrylamide copolymers and mixtures thereof. The preferred oxaziline is polyoxaziline.

The additional polymers which may be useful in the present invention comprise natural polymers selected from the group consisting of cellulose derivatives, algin and its derivatives, starch and its derivatives, guar and its derivatives, shellac polymers and mixtures thereof. Preferred cellulose derivatives are selected from the group consisting of hydroxyethylcellulose, hydroxypropylcellulose, hydroxypropyl methylcellulose, ethylhydroxyethyl cellulose and mixtures thereof.

## Fats

Fats employed according to the invention are selected from the group consisting of fats derived from animals, vegetables, synthetically derived fats, and mixtures thereof wherein said fats have a melting point from about 55°C to about 100°C and a needle penetration value, as measured according to the American standard ASTM D5, from about 3 to about 40 units at 25°C. Preferably the fats selected for use in the present invention are fatty acid esters which are solids at room temperature and exhibit crystalline structure. Examples of fatty acid esters useful in the present invention include the glyceryl esters of higher fatty acids such as stearic and palmitic such as glyceryl monostearate, glyceryl distearate, glyceryl tristearate, palmitate esters of glycerol, $C_{18-36}$ triglycerides, glyceryl tribehenate and mixtures thereof.

## Plasticizing solvents

Plasticizing solvents suitable for use herein are slow-evaporating, water-miscible or dispersible cosolvents that are 1) generally recognized as safe or 2) include slow evaporating glycols and glycol ethers, such as propylene glycol; butylene glycol; hexylene glycol; dipropylene glycol; dipropylene glycol methyl ether (commonly known as DPM); propylene glycol phenyl ether; and polyethylene glycols (PEGs) such as PEG 4 and PEG 8. Other exemplary plasticizing solvents include propylene carbonate, dimethyl isosorbide, and mixtures thereof. A wide variety of plasticizing solvents are listed in the CTFA International Cosmetic Ingredient Dictionary and Handbook, 3rd Ed., Cosmetic and Fragrance Assn., Inc., Washington D.C. (1982) pp. 575-580. The plasticizing solvent may be present in amounts of from 0.0% to 30% or even 5% to 20%, and generally appear in a ratio of solvent to polymer of from 10:1 to 1:5 or even 4:1 to 1:2. The plasticizing solvent is chosen to provide for water co-solvency, suitable solubility regarding the polymer, low volatility, stability, and safety (i.e., lack of toxicity). Thus, the cosmetic composition herein employs safe solvents that provide little or no sensation of tackiness or cooling (usually due to evaporation) on the applied area.

The plasticizing solvent may be chosen such that the polymer and plasticizing solvent are formulated in the aqueous phase of the emulsion, which may help reduce any tacky sensation of polymer contacting the user's hands and fingers during application of the cosmetic composition. Because the solvent exhibits a slow evaporation rate and is present in the aqueous phase, it helps extend the workability of the mascara and delays any perceived onset of tackiness for up to two minutes.

## Rheology Modifiers

Rheology modifiers that may be useful in the present invention include both associated and non-associated thickeners, including alkaline swellable, hydrophobic modified, polyurethane type thickeners and structuring agents.

Useful rheology modifiers include natural gums and extracts, modified (semi-synthetic) gums and extracts, hydrophilic natural and synthetic silicate and clay mineral agents, hydrophobic silicas, inorganic and polymeric porous microparticle absorbents, synthetic polymers (such as acrylic polymers), and mixtures thereof.

Natural gums and extracts of the present invention are selected from, but not limited to, the group consisting of plant exudates, such as gum arabic, gum tragacanth, gum karaya, and gum ghatti; plant extracts, such as pectins; plant seed flours or extracts, such as locust bean gum, guar gum, psyllium seed gum, and quince seed gum; seaweed extracts, such as agar, alginates, and carrageenans; seed starches, such as corn starch, wheat starch, rice starch, and sorghum starch; tuber starches, such as tapioca starch and potato starch; animal extracts, such as gelatin and caseinates; and mixtures thereof.

Modified (semi-synthetic) gums and extracts of the present invention are selected from, but not limited to, the group consisting of cellulose derivatives, such as sodium carboxymethylcellulose, hydroxyethylcellulose, hydroxypropylcellulose, methylcellulose, and hydroxypropyl methylcellulose, as well as alkyl-modified cellulose derivatives, such as cetyl hydroxyethylcellulose; modified plant extracts, such as hydroxypropyl guar; microbial or biosynthetic gums, such as xanthan gum, sclerotium gum, gellan gum, dextran and its derivatives; modified starches and starch derivatives, such as potato starch modified, corn starch modified, hydroxypropyl starch, dextrin and its derivatives; modified animal derivatives, such as chitin or chitosan, and their derivatives, collagen derivatives; and mixtures thereof.

Hydrophilic natural and synthetic clay mineral agents of the present invention are selected from, but not limited to, the group consisting of hectorites, such as those sold under tradenames BENTONE® (Elementis Specialties); bentonites and montmorillonites, such as those sold under tradenames OPTIGEL®, GELWHITE® and MINERAL COLLOID® (by BYK Additives & Instruments), and POLARGEL® (AMCOL Health & Beauty Solutions); magnesium aluminum silicates, such as those sold under tradenames VEEGUM® (R. T. Vanderbilt Company), MAGNABRITE® (AMCOL Health & Beauty Solutions), and GELWHITE® MAS (BYK); sodium magnesium silicate, such as those sold under tradenames OPTIGEL® SH and LAPONITE® (both by BYK); lithium magnesium sodium silicate, such as LUCENTITE® SWN (Kobo Products); lithium magnesium silicate, such as LUCENTITE® SAN (Kobo Products); and mixtures thereof.

Hydrophobic silicas of the present invention are selected from, but not limited to, the group consisting of hydrophobically modified fumed silicas, such as WACKER HDK® H15, H20, and H30 (Wacker-Chemie), and hydrophobic grades under tradenames of AEROSIL® (Degussa AG) and CAB-O-SIL® (Cabot Corporation); and mixtures thereof.

Inorganic and polymeric porous microparticle absorbents of the present invention are selected from, but not limited to, the group consisting of high porosity/void volume fumed silicas, such as MSS-5003H and Silica Shells (both sold by Kobo Products), high porosity/void volume silicates like calcium silicate, such as sold under tradename HUBERDERM™ (J. M. Huber Corporation); high porosity/void volume polymeric particle absorbents including methacrylate polymers like allyl methacrylates copolymer, sold as POLY-PORE® E-200 (AMCOL Health & Beauty Solutions), and cross-linked dimethacrylate copolymers like lauryl methacrylate/glycol dimethacrylate crosspolymer sold as POLYTRAP® 6603 (Enhanced Derm Technologies); high porosity cellulose beads like Cellulobeads® (Kobo Products); and mixtures thereof.

Synthetic polymers of the present invention include, but are not limited to, acrylic polymers, such as polyacrylates and polymethacrylates, and acrylic copolymers and crosspolymers, such as the carbomers or acrylates/C10-C30 alkyl acrylate crosspolymers sold under tradename CARBOPOL® (Lubrizol), and sodium polyacrylate sold under tradename RAPITHIX™ A-100 (ASI); alkali-soluble/swellable emulsion (ASE) polymers, hydrophobically-modified alkali-soluble/swellable emulsion (HASE) polymers, and hydrophobically-modified ethoxylated urethane (HEUR) polymers, such as those sold under tradename ACULYN™ (Dow Chemical Company) and STRUCTURE® (Akzo Nobel Company); hydrophobically-modified ethoxylate urethane alkali-soluble/swellable emulsion (HUERASE) polymers, such a those sold under tradename UCAR® POLYPHOBE® (Dow Chemical Company); copolymers of methyl vinyl ether and maleic anhydride, such as PVM/MA decadiene crosspolymer sold under tradename STABILEEZE® (ASI); hydrophobically modified non-ionic associative thickeners such as those sold under tradename PURE-THIX® (BYK); and mixtures thereof.

Oil Soluble or Oil Dispersible Additives

The choice of oil-soluble or dispersible additive and the amount present according to the invention will depend on the intended use of the composition and the effectiveness of the compound. In semi-permanent mascara, top coat and remover compositions, the oil-soluble or dispersible additive chosen is acceptable for skin and eye contact, as is well known to the skilled formulator. Suitable oil-soluble or dispersible additives are incorporated at levels generally between 1 and 20% by weight based on the weight of the matrix bead (equivalent to 90 to 300 % on weight of the colorant). Preferably 5 to 15% by weight of the oil-soluble or dispersible additive is employed.

The oil-soluble or dispersible additive may include fatty alcohols such as Guerbet alcohols based on fatty alcohols having from 6 to 30, preferably from 10 to 20 carbon atoms including lauryl alcohol, cetyl alcohol, stearyl alcohol, cetearyl alcohol, oleyl alcohol, benzoates of $C_{12}$-$C_{15}$ alcohols, acetylated lanolin alcohol, etc. Especially suitable is stearyl alcohol. The oil-soluble or dispersible additive may include fatty acids such as Linear fatty acids of $C_6$-$C_{24}$, branched

$C_6$-$C_{13}$carboxylic acids, hydroxycarboxylic acids, caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerization of unsaturated fatty acids). Further components that can be used are dicarboxylic acids of $C_2$-$C_{12}$, such as adipic acid, succinic acid, and maleic acid. Aromatic carboxylic acids, saturated and/or unsaturated, especially benzoic acid, can be used. Additional components that can be used as the oil soluble or dispersible additive include carboxylic acid salts; alkaline soaps of sodium, potassium and ammonium; metallic soaps of calcium or magnesium; organic basis soaps such as lauric, palmitic, stearic and oleic acid, etc., alkyl phosphates or phosphoric acid esters: acid phosphate, diethanolamine phosphate, potassium cetyl phosphate.

Other useful oil-soluble or dispersible additives comprise mild surfactants, super-fatting agents, consistency regulators, additional thickeners, polymers, stabilizers, biologically active ingredients, deodorizing active ingredients, anti-dandruff agents, film formers, swelling agents, UV light-protective factors, antioxidants, preservatives, insect repellents, solubilizers, colorants, bacteria-inhibiting agents, hair conditioning agents, vitamins, and the like.

Mascara Top Coat or Base Coat

It is to be appreciated that the mascara compositions described herein may be used in conjunction with another composition in a sequential application process. For example, the mascara composition may be used as a top coat or base coat in a multi-step mascara regimen. Suitable examples of top coats and base coats are described in copending U.S. Serial No. 13/274,852.

Applicator

Useful mascara applicators for use with the present invention include, but are not limited to, twisted-wire brush applicators and molded plastic brush type applicators. These applicators may be suitable, provided such applicators are able to suitably separate a user's eyelashes and minimize or even prevent clumping.

A suitable mascara product according to the present disclosure includes a container (e.g., bottle or the like) for storing a supply of the present mascara composition; a wiping system that cleans residual product from the applicator; an applicator for transferring mascara from the container to the eyelashes of a user; and a supply of the present mascara composition disposed in the container. The applicator may include a handle which is removably and/or reattachably joined to the container (e.g., with screw threads, snap collar, or the like).

TEST METHODS

Contraction Test

The principle of the contraction measurement is based on the shrinkage degree of a substrate after polymer compositions are applied to substrate and dried.

Equipment:

1. Leneta cards Form 2A (double coated opacity) with a dimension of 14cm x 25.4cm, supplied by Leneta Company.

2. Single bar 3-inch Film Applicator, 6 mils thickness, supplied by BYK Gardner

3. Drawdown Plate PA4200, supplied by BYK Gardner

4. Digital Humidity/Temperature Meter (Traceable® Model 35519-044 from VWR), or equivalent

5. Digital balance (with minimum sensitivity of at least 0.001 g)

6. Measuring ruler (30 cm with mm scaling)

Procedure:

1. Pre-weigh the Leneta card and record weight before a drawdown process. The drawdown method may refer ASTM D4062 or ASTM D2805 standard test methods.

2. Position and secure the Leneta card on the Drawdown Plate.

3. Place the bar film applicator centered at the top of the card, and load 5-10 grams of polymer compositions distributed evenly across and immediately in front of the bar applicator. Ensure the amount of polymer composition load is sufficient to pass over the end of the card that gives a covered area of about 3 inches x 8 inches by the casted film.

4. Drawdown uniformly in the center of the card all the way down, and pass the polymer compositions to the end of the card and onto the drawdown plate. Ensure the casted film is evenly distributed and in an essentially rectangular shape.

5. Allow the film to dry in a horizontal position for minimum 4 hrs (typically overnight).

6. Conduct experiments at a relative humidity of 40-50% and at a temperature in the range of 22°C to 28°C.

7. After the film is dried, the card is weighed again to determine the amount of total solids of the polymer compositions loaded by calculating the differences between the weights before and after the film cast.

Measurement and Calculation:

Depending on the type of contraction or curling effect observed for a given composition on a card, only one of the calculation formulas below should be selected for best evaluations of the contraction under specified relative humidity and temperature conditions.

1. On a flat, untreated Leneta card (such as shown in FIG. 1), measure the distances (measured to nearest tenth of cm) from the top edge to bottom edge of the card in both right (R) and left (L) sides of the card. As shown in FIG. 1, L is the length on the left side of the card and R is the length on the right side of the card. Then, for an evenly contracted card (such as shown in FIG. 2), measure the L and R lengths after treatment with a polymer composition and allowing the card to dry. The degree of contraction (% Contraction) of the polymer composition is calculated as follows:

$$\% \text{ Contraction} = 100 \text{ x } [1 - (R + L) / (25.4 \text{ x } 2)]$$

For example:
If L = 7.1 cm, and R = 6.9 cm of an evenly contracted card (such as shown in FIG. 2), the % contraction is calculated below:

$$\% \text{ Contraction} = 100 \text{ x } [1 - (6.9 + 7.1) / (25.4 \text{ x } 2)] = 72.4\%$$

2. For non-even contraction or twisted cards, such as shown in FIG. 3, measure the distances (measured to nearest tenth of cm) from the top edge to bottom edge of the card in both right (R) and left (L) sides of the card, and also the distances of the diagonal of the card from the right top to left bottom (RL) and from the left top to right bottom (LR). The degree of contraction (% Contraction) of the polymer composition is calculated as follows:

$$\% \text{ Contraction} = 100 \text{ x } [1 - (R + L + RL + LR) / (25.4 \text{ x } 2 + 29 \text{ x } 2)]$$

For example, if L = 5.8 cm, R = 2.9 cm, LR = 13.6, and RL = 15.2 of a non-evenly contracted twisted card, the % Contraction is calculated below:

$$\% \text{ Contraction} = 100 \text{ x } [1 - (5.8 + 2.9 + 13.6 + 15.2) / (25.4 \text{ x } 2 + 29 \text{ x } 2)] = 65.5\%$$

3. For coiled cards (such as shown in Fig. 4), measure diameters on both right (dR) and left (dL) edges (measure to nearest tenth of cm). The degree of contraction (% Contraction) of the card is calculated as follows:

$$\% \text{ Contraction} = 100 \text{ x } 3.4218 \text{ x } (1/dR + 1/dL)$$

For example, if dL = 7.2 cm and dR = 6.3 cm of a coiled card (where dL and dR are diameters measured from left side and right side respectively), the % Contraction is calculated below:

$$\% \text{ Contraction} = 100 \text{ x } 3.4218 \text{ x } (1/7.2 + 1/6.3) = 102\%$$

The Leneta card Contraction Test is conducted primarily for polymer technical screening. The Leneta card method can be used in combinations with other contraction methods for contraction performances of polymer systems. Other methods may include image analysis method for curling and eyelash/hair lifting on false lashes, image analysis method on human eye lashes, and consumer panel test.

Weight Loss Test (for Dry Speed evaluation)

The principle of the Dry Speed measurement is based on weight loss of the polymer compositions due to evaporation of volatile components (carrier/solvents) with time under specified conditions of relative humidity (%RH) and temperature.

Equipment:

1. Digital balance (Model AT460 4 decimal digits by Mettler Toledo with balance chamber enclosure), or equivalent
2. Air flow meter (Kontes by Granger), or equivalent
3. Dry nitrogen gas supply (Compressed) by Air Gas
4. Glass microscope slides (3-inch x 1-inch x 1-mm) from VWR
5. 7/8 inch hole Arch Punch
6. Hammer
7. Films: Bytac® type VF-81/FEP PTFE protection film (9 mil thickness) available from Saint Gobain-Performance Plastics (Item# 1435-AB)
8. Straight-edged scraper (Precision Gate & Tool A-1)
9. Digital timer

Procedure:

1) Prepare film template strips by cutting the Bytac protection films into about 1.5 inches x 2.5 inches size.
2) Punch a 7/8 inch hole centered at one end of the Bytac strip using the Arch Punch and hammer.
3) Turn on Nitrogen gas, and set automatic stop with defined time (usually about 2 hours).
4) Check and make sure gauge attached to balance chamber with flow meter reading at 1.1 liter per min.
5) Put a glass slide into the balance chamber and tare the glass slide weight on the balance, then remove glass slide from the balance.
6) Remove the protective layer from the back side of the Bytac film template and attach it evenly and carefully onto the glass slide with the hole positioned at the middle of the slide.
7) Press the film template with a clean straight-edged scraper up and down to remove air trapped under the film.
8) Load about 1 g of a polymer sample onto the top side of the hole.
9) Draw the polymer sample evenly across the hole on the glass slide using the straight-edged scraper to cover the hole area completely.
10) As soon as product is applied, peel the film template off the slide.

11) Immediately put the glass slide with product back to the balance, close the balance chamber door and start timing.
12) Record relative humidity (% RH) and temperature of the test condition.
13) The weight loss test will be automatically stopped when the weight loss reaches equilibrium if the balance is interfaced with a computer, or manually stopped when the recorded weight is no longer changed.
14) Record the weight changes for every 15-20 seconds until the weight loss has reached the equilibrium or minimum via any suitable computer software program or by manual recording.
15) Generate a drying profile graph of weight of the polymer composition against the time (in seconds) during the whole drying process based on the record.

Measurement and Calculation:

Based on the drying profile, determine 90% dry weight (as 90% total weight loss) and corresponding time.

90% dry weight = Starting Sample Wt. – 0.9 x [Starting Sample Wt. – Final Sample Wt. (at 60 minutes drying or at drying equilibrium)]

90% dry time = corresponding time (in minutes) taken to reach 90% dry weight

The Dry Speed of a polymer composition is defined as the 90% dry time, or time required to reach 90% total weight loss.

EXAMPLES

Following polymer combinations were prepared as 20% total polymer solids levels dissolved in deionized water:

Table 1: Sample List

| Sample # | Polymer-1 | Polymer-2 | Ratio of Polymer-1 solids to Polymer-2 solids |
|---|---|---|---|
| Sample 1 | AquaStyle 300[1] | Advantage S[2] | 1:1 (10%:10%) |
| Sample 2 | AquaStyle 300 | Copolymer 845[3] | 1:1 |
| Sample 3 | AquaStyle 300 | PVP K90[4] | 1:1 |
| Sample 4 | AquaStyle 300 | Ultrahold Strong[5] | 1:1 |
| Sample 5 | AquaStyle 300 | Aquaflex XL30[6] | 1:1 |
| Sample 6 | Advantage S | Copolymer 845 | 1:1 |
| Sample 7 | Advantage S | Luviquat Hold[7] | 1:1 |
| Sample 8 | Advantage S | Luviquat Supreme[8] | 1:1 |
| Sample 9 | Advantage S | Styleze W20[9] | 1:1 |
| Sample 10 | Styleze 2000[10] | AquaStyle 300 | 1:3 (5%:15%) |
| Sample 11 | Flexan II[11] | Styleze W20 | 1:3 |
| Sample 12 | Flexan II | Luviquat Hold | 1:3 |

[1]AquaStyle 300 is available from ASI (INCI name: Polyquaternium-69)
[2]Advantage S is available from ASI (INCI name: Vinylcaprolactam/VP/Dimethylaminoethyl Methacrylate Copolymer)
[3]Copolymer 845 is available from ASI (INCI name: VP/Dimethylaminoethyl Methacrylate Copolymer)
[4]PVP K90 is available from ASI (INCI name: PVP)
[5]Ultrahold Strong is available from BASF (INCI name: Acrylates/t-Butylacrylamide Copolymer)
[6]Aquaflex XL30 is available from ASI (INCI name: Polyimide-1)
[7]Luviquat Hold is available from BASF (INCI name: Polyquaternium-46)
[8]Luviquat Supreme is available from BASF (INCI name: Polyquaternium-68)
[9]Styleze W20 is available from ASI (INCI name: Polyquaternium-55)
[10]Styleze 2000 is available from ASI (INCI name: VP/Acrylate/Lauryl Methacrylate Copolymer)
[11]Flexan II is available from Akzo Nobel (INCI name: Sodium Polystyrene Sulfonate)

Mixing Procedure:

1. Polymer-1 and Polymer-2 combinations were mixed in a 200 mL jar in amounts to achieve polymer solids ratio as listed in the Table 1 above. QS with deionized water in jar to a proper amount to achieve target amount of 20% total polymer in mixture.

2. Manually stir carefully to remove lumps if any.

3. Homogenize the products in a SpeedMixer (by FlackTek Inc) until a homogeneous mixture is produced.

Evaluations:

The polymer combination was evaluated in comparison with each single polymer of that combination at equal total polymer levels (20% solid in the aqueous solutions or dispersions in the examples) with following methods:

1. Contraction Test
2. Weight Loss Test

As described in the Contraction Test, the % Contraction is calculated based on the distances measured from the top to bottom of the card on both the right (R) and the left (L) sides of the card for an evenly contracted card (as seen in Fig 2). For a non-even or twisted card, the % Contraction is calculated based on the diagonal measurements (RL and LR) and side measurements (R and L) of the card (as seen in Fig 3). For a coiled card, the % Contraction is calculated based on the diameter measurements for both the right and left sides (dR and dL) of the card (as seen in Fig 4).

As described in the Weight Loss Test, the Dry Speed is determined as the time when 90% of total weight loss by solvent evaporation is achieved.

The "dry speed synergy" of a polymer combination is calculated based on the following equations:

$$S = \frac{100\ \mathrm{C}\ (x+y)}{(\mathrm{Ax+By})} \text{ for Synergy of Contraction}$$

$$S = \frac{100\ (\mathrm{Ax+By})}{\mathrm{C}\ (x+y)} \text{ for Synergy of Dry Speed}$$

where C is the resulting value of Contraction (in %) or Dry Speed (in minutes) associated with a polymer combination, and A and B are the resulting values of Contraction (in %) or Dry Speed (in minutes) associated with the corresponding Polymer-1 and Polymer-2 of that combination, respectively, and x represents the proportion of Polymer-1 solid, and y represents the proportion of Polymer-2 solid, while x : y is the ratio of Polymer-1 to Polymer-2 solids, expressed as:

$$\text{x} = \text{\% Polymer-1 solids in sample / (\% Total Polymer-1 + Polymer-2 solids in sample)}$$

$$\text{y} = \text{\% Polymer-2 solids in sample / (\% Total Polymer-1 + Polymer-2 solids in sample)}$$

For example, in a Contraction Test, a single Polymer-1 (A), a single Polymer-2 (B), and a combination of Polymer-1 and Polymer-2 (C) in a ratio of 2:3 (x:y) provide % Contraction of 10% (A), 20% (B), and 25% (C) respectively. The synergy S of combination is calculated below:

$$S = \frac{100\ \mathrm{C}\ (\mathrm{x+y})}{(\mathrm{Ax+By})} = 100 \text{ x } 25\% \text{ x } (2 + 3) / (10\% \text{ x } 2 + 20\% \text{ x } 3) = 156\%$$

For example, in a Weight Loss Test, a single Polymer-1, a single Polymer-2, and a combination of Polymer-1 and Polymer-2 in a ratio of 2:3 (x:y) provide Dry Speed of 10 minutes (A), 20 minutes (B), and 12 minutes (C), respectively.

The synergy S of combination is calculated below:

$$S = \frac{100\ (\mathrm{Ax+By})}{\mathrm{C}\ (x+y)} = 100 \text{ x } (10 \text{ x } 2 + 20 \text{ x } 3) \ / \ 12 \text{ x } (2 + 3) = 133\%$$

The results of the film Dry Speed based on the Weight Loss (in minutes) and Contraction (in %) as well as degree of positive or negative synergy (in %) of the polymer combinations are summarized in the tables below.

Table 2: Results of Dry Speed (at 90% based on Weight Loss Test) and Contraction (at 45 - 47% RH and 25±3°C on Leneta card) with single polymers and polymer combinations at total polymer solids of 20% where Positive synergy: >110%, Negative synergy: <90%, and no synergy: 90% - 110%

| | Dry Speed (in minute) | Synergy of Dry Speed | Contraction | Synergy of Contraction |
|---|---|---|---|---|
| Aquastyle 300 | 8.54 | | 29.10% | |
| Advantage S | 9.15 | | 0.00% | |
| Sample 1 (from Table 1) | 7.00 | 126% | 74.41% | 511% |
| Aquastyle 300 | 8.54 | | 29.10% | |
| Copolymer 845 | 50.63 | | 0.00% | |
| Sample 2 (from Table 1) | 7.02 | 421% | 1.18% | 8% |
| Aquastyle 300 | 8.54 | | 29.10% | |
| PVP K90 | 12.20 | | 0.00% | |
| Sample 3 (from Table 1) | 10.37 | 100% | 0.98% | 7% |
| Aquastyle 300 | 8.54 | | 29.10% | |
| Ultrahold Strong | 5.80 | | 0.00% | |
| Sample 4 (from Table 1) | 5.80 | 124% | 1.38% | 9% |
| Aquastyle 300 | 8.54 | | 29.10% | |
| Aquaflex XL30 | 11.29 | | 0.00% | |
| Sample 5 (from Table 1) | 17.08 | 58% | 1.60% | 11% |
| Advantage S | 9.15 | | 0.00% | |
| Copolymer 845 | 50.63 | | 0.00% | |
| Sample 6 (from Table 1) | 8.24 | 363% | 0.00% | N/A |
| Advantage S | 9.15 | | 0.00% | |
| Luviquat Hold | 8.85 | | 0.79% | |
| Sample 7 (from Table 1) | 14.64 | 61% | 1.57% | 397% |
| Advantage S | 9.15 | | 0.00% | |
| Luviquat Supreme | 10.98 | | 1.18% | |
| Sample 8 (from Table 1) | 14.95 | 67% | 11.02% | 1868% |

(continued)

| | Dry Speed (in minute) | Synergy of Dry Speed | Contraction | Synergy of Contraction |
|---|---|---|---|---|
| Advantage S | 9.15 | | 0.00% | |
| Styleze W20 | 12.81 | | 0.00% | |
| Sample 9 (from Table 1) | 14.34 | 77% | 0.00% | N/A |
| Aquastyle 300 | 8.54 | | 29.10% | |
| Styleze 2000 | 9.46 | | 0.40% | |
| Sample 10 (from Table 1) | 10.68 | 82% | 1.90% | 13% |
| Styleze W20 | 12.81 | | 0.00% | |
| Flexan II | 50.33 | | 0.00% | |
| Sample 11 (from Table 1) | 13.42 | 165% | 1.60% | N/A |
| Luviquat Hold | 8.85 | | 0.79% | |
| Flexan II | 50.33 | | 0.00% | |
| Sample 12 (from Table 1) | 9.15 | 210% | 3.60 % | 608% |

Table 3: Effect of glycerin levels on Dry Speed (at 90% based on Weight Loss Test) and Contraction (50% RH and 25±3°C on Leneta card) in a film-forming composition (10% Advantage S polymer solids, 10% Aquastyle 300 polymer solids, 10% ethanol, 1% beeswax, and glycerin at different levels with QS deionized water, as shown in the table below)

| | Dry Speed (in minutes) | Contraction (%) |
|---|---|---|
| 0% glycerin | 8.24 | 100% |
| 0.5% glycerin | 10.37 | 57% |
| 2.0% glycerin | 10.37 | 32% |
| 5.0% glycerin | 10.68 | 0% |

## Claims

**1.** A film forming composition comprising:

a. a first non-crosslinking polyamide/polyacrylate copolymer comprising the following monomer units:

i. at least one amide monomer, including vinyl caprolactam monomers, vinylpyrrolidone monomers, and acrylamide monomers;
ii. (meth)acrylate monomers;
iii. monomers having at least one carboxylic functional group selected from the group consisting of carboxylic esters, carboxylic acids, their salts, or precursors of carboxylate functions, and mixtures thereof; and
iv. monomers having at least one amine functional group including primary, secondary and tertiary amines

b. a second non-crosslinking polyamide copolymer comprising the following monomer units:

i. at least one amide monomer, including vinyl caprolactam monomers, vinylpyrrolidone monomers, and (meth)acrylamide monomers;
ii. at least one quaternary ammonium containing monomer and

iii. monomers having at least one amine functional group including primary, secondary, and tertiary amines;

wherein the film forming composition comprises less than 0.5 weight % of glycerin; and wherein said first and second copolymers comprise from about 40% to about 99% of identical monomer units.

2. The composition of claim 1 wherein said first and second copolymers have from about 60% to about 99% of identical monomer units.

3. The composition of claim 1 wherein said first and second copolymers have from about 70% to about 99% of identical monomer units.

4. The composition of claim 1 wherein said second copolymer has one or more quaternary ammonium or other cationic side chains and further, wherein said second polymer comprises from about 0.1 to about 45 percent of said quaternary ammonium or other cationic side chains.

5. The composition of claim 1 wherein said first copolymer has one or more carboxylic or other anionic side chains and further, wherein said first polymer comprises from about 0.1 to about 45 percent of said carboxylic or other anionic side chains.

6. The composition of claim 1 wherein the ratio of said first copolymer to said second copolymer is from about 1:10 to about 10:1.

7. The composition of claim 1 wherein the ratio of said first copolymer to said second copolymer is about 1:3 to about 3:1.

8. The composition of claim 1 wherein said first and second copolymers have a combined active polymer level greater than about 1%.

9. The composition of claim 1 wherein said first and second copolymers each have a combined active polymer level from about 5% to about 30%.

10. The composition of claim 1 wherein said first and second copolymers are random copolymers.

11. A water based system comprising the film forming composition of claim 1.

12. A mascara formulation comprising the composition of claim 1.

13. A skin foundation product comprising the composition of claim 1.

14. The composition of claim 1, wherein the composition is in the form of a dry film, a dry powder, or combinations thereof.

## Patentansprüche

1. Filmbildende Zusammensetzung, umfassend:

   a. ein erstes nichtvernetzendes Polyamid/Polyacrylat-Copolymer, umfassend die folgenden Monomereinheiten:

      i. mindestens ein Amidmonomer, einschließlich Vinylcaprolactam-Monomeren, Vinylpyrrolidon-Monomeren und Acrylamid-Monomeren;
      ii. (Meth)acrylat-Monomere;
      iii. Monomere mit mindestens einer carboxylfunktionellen Gruppe, ausgewählt aus der Gruppe, bestehend aus Carbonsäureestern, Carbonsäuren, ihren Salzen oder Vorstufen von Carboxylatfunktionen und Gemischen davon; und
      iv. Monomere mit mindestens einer aminfunktionellen Gruppe, einschließlich primärer, sekundärer und tertiärer Amine;

   b. ein zweites nichtvernetzendes Polyamid-Copolymer, umfassend die folgenden Monomereinheiten:

i. mindestens ein Amidmonomer, einschließlich Vinylcaprolactam-Monomeren, Vinylpyrrolidon-Monomeren und (Meth)acrylamid-Monomeren;
ii. mindestens ein quartäres Ammonium enthaltendes Monomer und
iii. Monomere mit mindestens einer aminfunktionellen Gruppe, einschließlich primärer, sekundärer und tertiärer Amine;

wobei die filmbildende Zusammensetzung weniger als 0,5 Gewichts-% Glycerin umfasst; und wobei das erste und das zweite Copolymer zu etwa 40 % bis etwa 99 % identische Monomereinheiten umfassen.

**2.** Zusammensetzung nach Anspruch 1, wobei das erste und das zweite Copolymer zu etwa 60 % bis etwa 99 % identische Monomereinheiten aufweisen.

**3.** Zusammensetzung nach Anspruch 1, wobei das erste und das zweite Copolymer zu etwa 70 % bis etwa 99 % identische Monomereinheiten aufweisen.

**4.** Zusammensetzung nach Anspruch 1, wobei das zweite Copolymer ein oder mehrere quartäre Ammonium- oder andere kationische Seitenketten aufweist und ferner, wobei das zweite Polymer zu etwa 0,1 bis etwa 45 Prozent die quartären Ammonium- oder anderen kationischen Seitenketten umfasst.

**5.** Zusammensetzung nach Anspruch 1, wobei das erste Copolymer eine oder mehrere carboxylische oder andere anionische Seitenketten aufweist und ferner, wobei das erste Polymer zu etwa 0,1 bis etwa 45 Prozent die carboxylischen oder anderen anionischen Seitenketten umfasst.

**6.** Zusammensetzung nach Anspruch 1, wobei das Verhältnis von dem ersten Copolymer zu dem zweiten Copolymer etwa 1 : 10 bis etwa 10 : 1 beträgt.

**7.** Zusammensetzung nach Anspruch 1, wobei das Verhältnis von dem ersten Copolymer zu dem zweiten Copolymer etwa 1 : 3 bis etwa 3 : 1 beträgt.

**8.** Zusammensetzung nach Anspruch 1, wobei das erste und das zweite Copolymer einen kombinierten Gehalt an aktivem Polymer von größer als etwa 1 % aufweisen.

**9.** Zusammensetzung nach Anspruch 1, wobei das erste und das zweite Copolymer jeweils einen kombinierten Gehalt an aktivem Polymer von etwa 5 % bis etwa 30 % aufweisen.

**10.** Zusammensetzung nach Anspruch 1, wobei das erste und das zweite Copolymer statistische Copolymere sind.

**11.** Wasserbasiertes System, umfassend die filmbildende Zusammensetzung nach Anspruch 1.

**12.** Mascaraformulierung, umfassend die Zusammensetzung nach Anspruch 1.

**13.** Hautgrundierungsprodukt, umfassend die Zusammensetzung nach Anspruch 1.

**14.** Zusammensetzung nach Anspruch 1, wobei die Zusammensetzung in Form eines trockenen Filmes, eines trockenen Pulvers oder von Kombinationen davon vorliegt.

## Revendications

**1.** Composition filmogène comprenant :

a. un premier copolymère de polyamide/polyacrylate non réticulé comprenant les unités monomères suivantes :

i. au moins un monomère d’amide, comprenant les monomères de vinylcaprolactame, les monomères de vinylpyrrolidone et les monomères d’acrylamide ;
ii. des monomères de (méth)acrylate ;
iii. des monomères ayant au moins un groupe fonctionnel carboxylique choisi dans le groupe constitué par les esters carboxyliques, les acides carboxyliques, leurs sels, ou les précurseurs de fonctions carboxylate,

et leurs mélanges ; et

iv. des monomères ayant au moins un groupe fonctionnel amine, comprenant les amines primaires, secondaires et tertiaires ;

b. un deuxième copolymère de polyamide non réticulé comprenant les unités monomères suivantes :

i. au moins un monomère d'amide, comprenant les monomères de vinylcaprolactame, les monomères de vinylpyrrolidone et les monomères de (méth)acrylamide ;

ii. au moins un monomère contenant un ammonium quaternaire, et

iii. des monomères ayant au moins un groupe fonctionnel amine, comprenant les amines primaires, secondaires et tertiaires ;

ladite composition filmogène comprenant moins de 0,5 % en poids de glycérine ; et lesdits premier et deuxième copolymères étant constitués d'environ 40 % à environ 99 % d'unités monomères identiques.

**2.** Composition selon la revendication 1, dans laquelle lesdits premier et deuxième copolymères présentent environ 60 % à environ 99 % d'unités monomères identiques.

**3.** Composition selon la revendication 1, dans laquelle lesdits premier et deuxième copolymères présentent environ 70 % à environ 99 % d'unités monomères identiques.

**4.** Composition selon la revendication 1, dans laquelle ledit deuxième copolymère présente au moins une chaîne latérale d'ammonium quaternaire ou autrement cationique, et en outre dans laquelle ledit deuxième polymère présente environ 0,1 à environ 45 pour cent desdites chaînes latérales d'ammonium quaternaire ou autrement cationiques.

**5.** Composition selon la revendication 1, dans laquelle ledit premier copolymère présente au moins une chaîne latérale carboxylique ou autrement anionique, et en outre dans laquelle ledit premier polymère présente environ 0,1 à environ 45 pour cent desdites chaînes latérales carboxyliques ou autrement anioniques.

**6.** Composition selon la revendication 1, dans laquelle le rapport entre ledit premier copolymère et ledit deuxième copolymère est d'environ 1:10 à environ 10:1.

**7.** Composition selon la revendication 1, dans laquelle le rapport entre ledit premier copolymère et ledit deuxième copolymère est d'environ 1:3 à environ 3:1.

**8.** Composition selon la revendication 1, dans laquelle lesdits premier et deuxième copolymères ont un niveau de polymère actif combiné supérieur à environ 1 %.

**9.** Composition selon la revendication 1, dans laquelle lesdits premier et deuxième copolymères ont chacun un niveau de polymère actif combiné d'environ 5 % à environ 30 %.

**10.** Composition selon la revendication 1, dans laquelle lesdits premier et deuxième copolymères sont des copolymères aléatoires.

**11.** Système à base aqueuse comprenant la composition filmogène selon la revendication 1.

**12.** Formulation de mascara comprenant la composition selon la revendication 1.

**13.** Produit de fond de teint comprenant la composition selon la revendication 1.

**14.** Composition selon la revendication 1, laquelle se présente sous la forme d'un film sec, d'une poudre sèche ou de leurs combinaisons.

Fig 1: Length measurement for Leneta card % contraction

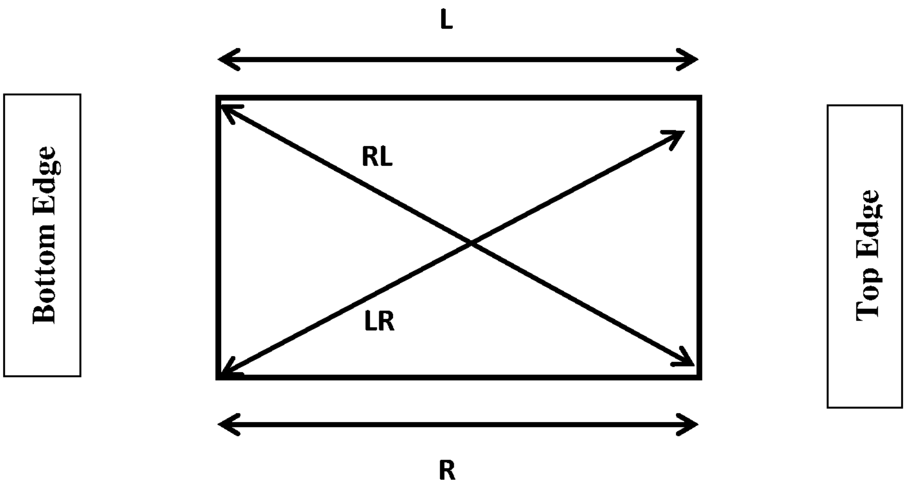


Fig 2: Contraction measurement on an evenly contracted card

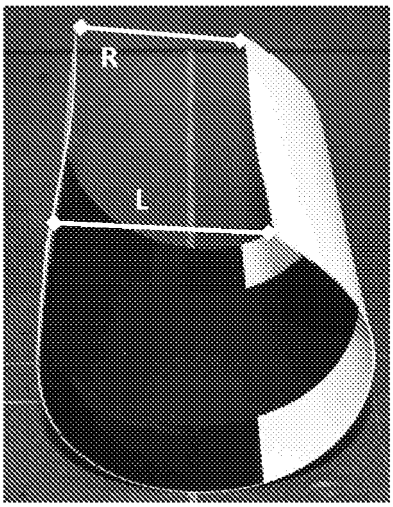

Fig 3: Contraction measurement on a non-even or twisted card

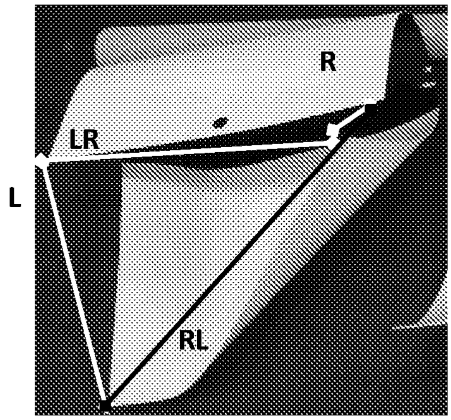


Fig 4: Contraction measurement on a coiled card

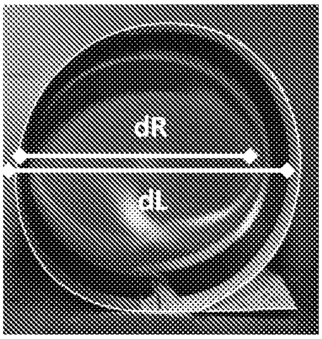


Fig 5: Curl Image of false lashes with application of a polymer combination having positive Synergy of Contraction

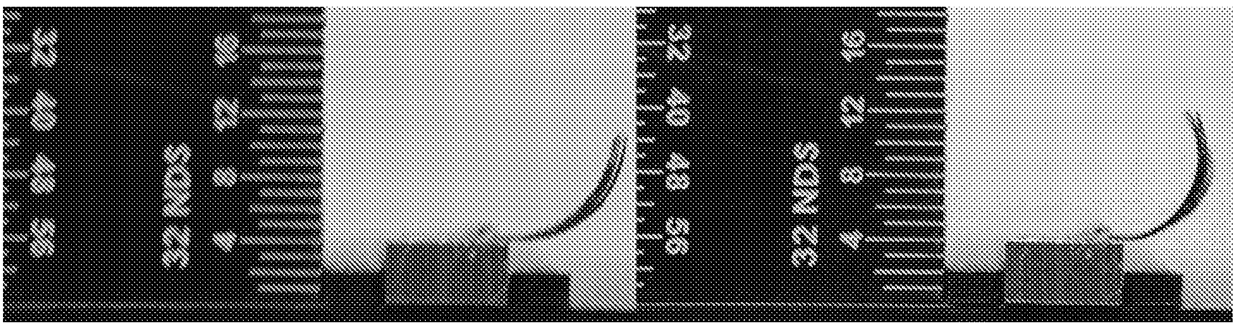

Before polymer application    After polymer application on top and drying

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009086338 A1
- US 6852815 B
- US 4781917 A
- US 20090263658 A
- US 20090271932 A1
- US 274852


### Non-patent literature cited in the description


- **TODD et al.** Volatile Silicone Fluids for Cosmetics. *Cosmetics and Toiletries,* 1976, vol. 91, 27-32
- Cosmetics, Science, and Technology. 1972, vol. 1, 27-104
- the PCPC Cosmetic Ingredient Handbook. Cosmetics, Toiletry and Fragrances Association, Inc, 1988
- CTFA International Cosmetic Ingredient Dictionary and Handbook. Cosmetic and Fragrance Assn., Inc, 1982, 575-580